# FASCICULE DE BREVET EUROPEEN

(11) **EP 1 023 320 B1**
(45) Date de publication et mention de la délivrance du brevet: **25.05.2005**
(21) Numéro de dépôt: 98950143.2
(22) Date de dépôt: 15.10.1998
(51) Int. Cl.: C07K 14/155, A61K 39/21

(54) **PEPTIDES ISSUS DE GENE ENV DU VIRUS DE L'IMMUNODEFICIENCE FELINE ET LEURS APPLICATIONS**
PEPTIDE DES ENV-GENS VOM KATZE IMMUNSCHWÄCHE-VIRUS UND DEREN VERWENDUNG
PEPTIDES DERIVED FROM THE env GENE OF THE FELINE IMMUNODEFICIENCY VIRUS AND THEIR APPLICATIONS

(30) Priorité: 17.10.1997 FR 9713042
(43) Date de publication de la demande: 02.08.2000
(73) Titulaire: Centre National de la Recherche Scientifique, 75794 Paris Cedex 16 (FR)
(72) Inventeur: RICHARDSON, Jennifer, F-91400 Orsay (FR); MORAILLON, Anne, F-94210 La Varenne Saint Hilaire (FR); SONIGO, Pierre, F-75015 Paris (FR); PANCINO, Gianfranco, F-75019 Paris (FR)
(74) Mandataire: Vialle-Presles, Marie José
(86) Numéro de dépôt international: PCT/FR1998/002213
(87) Numéro de publication internationale: WO 1999/020650

(56) Documents cités:
- EP-A- 0 577 458
- WO-A-93/08836
- WO-A-94/06471
- FR-A- 2 669 338
- RICHARDSON J ET AL.: "Neutralization sensitivity and accessibility of continuous B cell epitopes of the feline immunodeficiency virus envelope" JOURNAL OF GENERAL VIROLOGY., vol. 77, no. 4, 1996, pages 759-777, XP002041747 READING GB cité dans la demande
- RICHARDSON J ET AL: "Delayed infection after immunization with a peptide from the transmembrane glycoprotein of the feline immunodeficiency virus" JOURNAL OF VIROLOGY., vol. 72, no. 3, 1998, pages 2406-2415, XP002094008 US

## Description

La présente invention est relative à des peptides issus du gène *env* de VIF (virus de l'immunodéficience féline) ainsi qu'à leurs applications immunoprotectrices (prévention et traitement de l'immunodéficience féline).

L'immunodéficience féline est due à un lentivirus, le virus de l'immunodéficience féline (VIF), qui présente une structure génétique similaire à celle des lentivirus des primates (VIH et VIS).

Un certain nombre de fragments ont été sélectionnés et ont permis la mise au point de tests sensibles et spécifiques pour la détection des animaux séropositifs, comme décrits dans les Demandes de Brevets européens n° 0 564 477 du 20 novembre 1991, n° 0 577 458 du 16 juin 1993, et français n° 94 07062 du 9 juin 1994, au nom de la Demanderesse, et sont issus, pour la plupart de la protéine Env de VIF, comprenant 854 aminoacides, dont la séquence est décrite dans la Demande de Brevet européen n° 0 577 458.

La protéine Env fournit, après clivage, 2 fragments glycoprotéiques dénommés SU (glycoprotéine de surface) et TM (glycoprotéine transmembranaire), dans lesquels neuf domaines, comprenant des épitopes B continus des glycoprotéines d'enveloppe des VIF et reconnus lors de l'infection naturelle (Pancino G. et al., J. Virol, 1993, 67, 664-672), ont été définis.

Parmi ces neuf domaines, cinq (SU1-SU5) d'entre eux ont été localisés au niveau de la glycoprotéine de surface et quatre d'entre eux (TM1-TM4) au niveau de la glycoprotéine transmembranaire.

De manière plus précise, les positions des domaines TM1-TM4 dans la protéine TM sont les suivantes :
- le domaine TM1 (51 aminoacides) correspond aux positions 595-647 de la protéine Env de VIF;
- le domaine TM2 (31 aminoacides) correspond aux positions 681-711 de la protéine Env de VIF ; ce domaine contient un épitope incluant la séquence : Cys⁶⁹⁷-Asn-Gln-Asn-Gln-Phe-Phe-Cys-Lys⁷⁰⁵ (peptide dénommé P237),
- le domaine TM3 (45 aminoacides) correspond aux positions 744-788 de la protéine Env de VIF, et
- le domaine TM4 (29 aminoacides) correspond aux positions 826-854 de la protéine Env de VIF.

Alors que dans le domaine de la détection, l'on dispose maintenant d'un ensemble de réactifs pour la détection du VIF, dans le domaine de l'immunoprotection, la situation est plus complexe.

Le peptide TM2, par exemple, peut induire la formation d'anticorps facilitants, qui ont un effet contraire à celui recherché, à savoir un effet d'amplification de l'infection virale au lieu d'avoir un effet de protection.

En particulier, on a observé qu'une vaccination avec des préparations d'enveloppe du VIF entraîne une accélération ou une aggravation clinique de l'infection virale.

Bien qu'il soit habituellement reconnu que la fonction des anticorps est conventionnellement estimée par des essais de neutralisation de l'infectivité virale de cellules en culture, les résultats observés, ont montré que la plupart des anticorps neutralisants sont dirigés contre les glycoprotéines d'enveloppe, mais qu'une vaccination avec l'enveloppe lentivirale, malgré l'induction d'anticorps neutralisants, ne permet pas d'obtenir une protection convenable vis-à-vis d'infections expérimentales (Johnson R.P., *Curr. Opinion in Immunol.,* 1996, **8,** 554-560).

En particulier, on a observé, après une immunisation avec la glycoprotéine d'enveloppe du VIF et une épreuve virulente, soit une diminution de la charge virale (Hosie M.J. et al., *Vaccine,* 1996, **14**, 405-411), soit une aggravation de l'infection primaire (Siebelink K.H. et al., *J. Virol.,* 1995, **69,** 3, 3704-3711) chez les chats. Dans cette dernière étude (Siebelink K.H. et al., 1995, précité), l'aggravation de l'infection par le VIF s'observe également chez les animaux soumis à un transfert passif de plasma de chats immunisés ; de tels résultats évoquent la possibilité que ce sont les anticorps qui induisent l'augmentation de l'infection observée *in vivo*.

La possibilité que la réponse immune dirigée contre l'enveloppe du VIF soit délétère pour l'hôte a été confirmée par les Inventeurs, dans une expérience de vaccination par le gène *env,* qui a conduit à une accélération de l'infection.

Toutefois, aussi bien l'existence d'un lien de causalité entre l'aggravation de l'infection et la présence d'anticorps, que l'existence d'un lien entre le degré de protection obtenu et la présence d'anticorps sont difficiles à établir et montrent la complexité rencontrée pour mettre au point des compositions effectivement protectrices vis-à-vis des lentivirus et plus particulièrement du VIF. En effet, la présence d'anticorps facilitants dans le sérum des patients infectés par le VIH-1 a été corrélée, dans certaines études, avec la progression de la maladie (Fust G. et al., *AIDS,* 1994, **8**, 603-609 ; Homsy J. et al., *J. Virol*., 1990, **64**, 1437-1440).

Il est en particulier fort possible que les anticorps qui augmentent l'infectivité lentivirale, puissent également diminuer le degré d'immunité protectrice obtenue lors de l'infection naturelle, après vaccination avec des sous-unités de protéine d'enveloppe.

Bien que l'induction d'anticorps qui neutralisent l'infection virale *in vitro* ait été considérée jusqu'à présent comme un bon indicateur pour sélectionner les séquences aptes à induire une immunité protectrice, les résultats précités montrent qu'en fait il n'existe pas de corrélation activité neutralisante/protection (Matteuci D. et al., *J. Virol*., 1996, **70**, 617-622).

En outre, les conditions de mesure de l'activité et le choix des virus sur lesquels les tests sont effectués interviennent dans l'interprétation des résultats.

On peut citer comme paramètres particulièrement influents : le substrat cellulaire utilisé pour mesurer l'infectivité résiduelle et les passages du virus, l'utilisation d'isolats primaires par rapport aux virus adaptés au laboratoire.

La sélection d'anticorps monoclonaux à haut pouvoir neutralisant et dirigés contre des épitopes conformationnels a suggéré que des épitopes discontinus sont importants pour la protection. Mais l'identification et la synthèse de mimotopes pour l'utilisation vaccinale sont particulièrement difficiles à obtenir.

Des peptides, représentant des épitopes continus sont un réactif tout à fait adapté à des fins vaccinales. L'épitope continu neutralisant de VIH-1 le plus étudié est le principal domaine neutralisant de la glycoprotéine de surface, le domaine V3 (Goudsmit J. et al., *Proc. Natl. Acad. Sci*. USA, 1988, **85**, 4478-4482 ; Palker T.J. et al., *Proc. Natl. Acad. Sci*. USA, 1988, **85**, 1932-1936 ; Rusche J.R. et al., *Proc. Natl. Acad. Sci*. USA, 1988, **85**, 3198-3202). Toutefois, cette région est hypervariable et la neutralisation est limitée essentiellement au virus homologue.

Un peptide dérivé de la région V3 du VIF, qui induit des anticorps neutralisants, n'a pas induit une protection antivirale chez des chats immunisés (Lombardi, *J. Virol*., 1994, **68**, 8374-8379). Ceci montre bien que la neutralisation *in vitro* ne signifie pas directement capacité protectrice.

Compte tenu de la complexité de la réactivité du système immunitaire vis-à-vis des protéines lentivirales et du fait que tous les anticorps neutralisants n'ont pas systématiquement une activité protectrice, les Inventeurs se sont donné pour but d'utiliser des peptides synthétiques tels que ceux décrits dans la Demande de Brevet européen n° 0 577 458 pour préparer un médicament apte à enduire un certain degré de protection contre une infection à VIF et de sélectionner de nouveaux peptides synthétiques, aptes à induire un certain degré de protection contre une épreuve virulente avec une souche primaire de VIF ; une telle protection est observée par la diminution ou la suppression de la charge virale pendant l'infection aiguë.

La présente invention a pour objet l'utilisation du peptide de 19 aminoacides de :
- SEQ ID NO:1 ci-après : Lys-Lys-Gly-Leu-Gln-Gln-Leu-Gln-Glu-Trp-Glu-Asp-Trp-Val-Gly-Trp-Ile-Gly-Asn (SEQ ID NO:1)
pour préparer un médicament pour le traitement ou la prévention des infections à VIF.

La présente invention a également pour objet le peptide de SEQ ID NO:1, apte à induire un certain degré de protection (préventif ou thérapeutique) contre une infection à VIF.

De manière surprenante, on observe une diminution ou une suppression de la charge virale lors d'une infection aiguë, uniquement après immunisation avec le peptide de SEQ ID NO:1.

Le peptide de 19 aminoacides de SEQ ID NO:1 également dénommé TM3-19 est un fragment du peptide correspondant au domaine TM3.

Une analyse systématique de l'activité fonctionnelle des anticorps dirigés contre les différents domaines précités (SU1-SU5 et TM1-TM4) n'a permis d'identifier qu'un seul domaine, SU2, localisé dans la troisième région variable, comme étant capable d'induire des anticorps qui neutralisent l'infectivité virale de cellules en culture (de Ronde A. et al., *Virology,* 1994, **198**, 257-264 ; Lombardi S. et al., *J. Virol.,* 1993, **67**, 4742-4749 ; Richardson J. et al., *J. Gen. Virol*., 1996, **77**, 759-771).

Trois domaines (SU2, SU5 et TM3-19), reconnus par la majorité des chats infectés, ont été sélectionnés par les Inventeurs comme « candidats » comme immunogènes. Toutefois, bien que le domaine SU2 induise la production d'anticorps neutralisants, seul le fragment contenant la séquence SEQ ID NO:1 est effectivement protecteur vis-à-vis de l'infection.

De manière surprenante, la séquence TM3-19 ou les séquences de moins de 50 aminoacides la contenant, présente une activité protectrice (aussi bien préventive que curative), bien qu'elle n'induise pas la production d'anticorps neutralisants.

La présente invention a également pour objet une composition immunoprotectrice et/ou vaccinale contre une infection à VIF, caractérisée en ce qu'elle est essentiellement constituée par au moins le peptide de SEQ ID NO: 1 et au moins un véhicule pharmaceutiquement acceptable.

La présente invention a également pour objet une composition immunoprotectrice, telle que définie ci-dessus, pour son utilisation dans la prévention (vaccin) ou le traitement des infections à VIF.

L'évaluation de la charge virale en VIF et/ou de surveillance de l'efficacité de la vaccination ou du traitement dans un échantillon biologique peut être réalisée par une méthode qui comprend :
(a) l'extraction de l'ARN viral à partir d'un échantillon biologique à analyser ;
(b) la préparation d'une gamme d'échantillons contenant chacun un nombre différent d'un ARN de compétition, obtenu à partir d'une région conservée du gène *gag*, dans laquelle au moins une modification a été introduite ;
(c) le mélange de l'extrait d'ARN obtenu en (a) avec chaque échantillon de la gamme obtenue en (b), séparément ;
(d) la transcription inverse des ARN présents dans les différents mélanges obtenus en (c), pour obtenir l'ADNc correspondant ;
(e) l'amplification des ADNc obtenus en (d), à l'aide des amorces sélectionnées dans le groupe constitué par les séquences SEQ ID NO:7, SEQ ID NO:8, SEQ ID NO:9 et SEQ ID NO:10 ;
(f) la séparation des produits d'amplification et
(g) la détection du nombre de copies d'ARN viral initial par analyse quantitative convenable, telle que la densitométrie comparée et établissement du rapport densité de produit de l'ARN de compétition/densité de produit de l'ARN sauvage présent dans l'échantillon initial en fonction du nombre de copies d'ARN de compétition ajouté à chaque mélange.

L'ARN de compétition selon l'étape (b) est obtenu, dans le cas où ladite modification est une délétion, par :
(i) amplification de ladite région conservée à l'aide des amorces de séquence SEQ ID NO:4 et SEQ ID NO:5,
(ii) réalisation de la délétion par mutagénèse par PCR de la portion 3' du produit obtenu en (i), à l'aide des amorces de séquence SEQ ID NO:6 et SEQ ID NO:5,
(iii) remplacement de la portion 3' terminale du produit obtenu en (i) par le fragment portant la délétion obtenue en (ii), et
(iv) production dudit ARN de compétition en utilisant le produit final obtenu en (iii), comme matrice.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

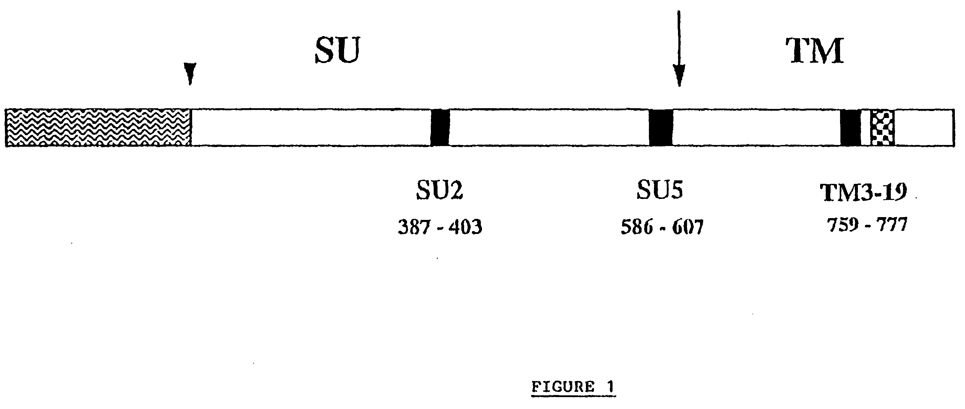

- la figure 1 est une représentation sous forme de diagramme des glycoprotéines d'enveloppe de VIF de surface (SU) et transmembranaire (TM). Les peptides SU2, SU5 et TM3-19 (-■-) sont utilisés comme immunogènes. Les sites de clivage protéolytique éliminant le peptide signal (◆) et générant les glycoprotéines d'enveloppe matures (↓) sont indiqués. Les positions correspondent à celles des glycoprotéines d'enveloppe de la souche Wo. La zone -□- correspond au domaine intermembranaire ; la zone -□- correspond au peptide signal ;

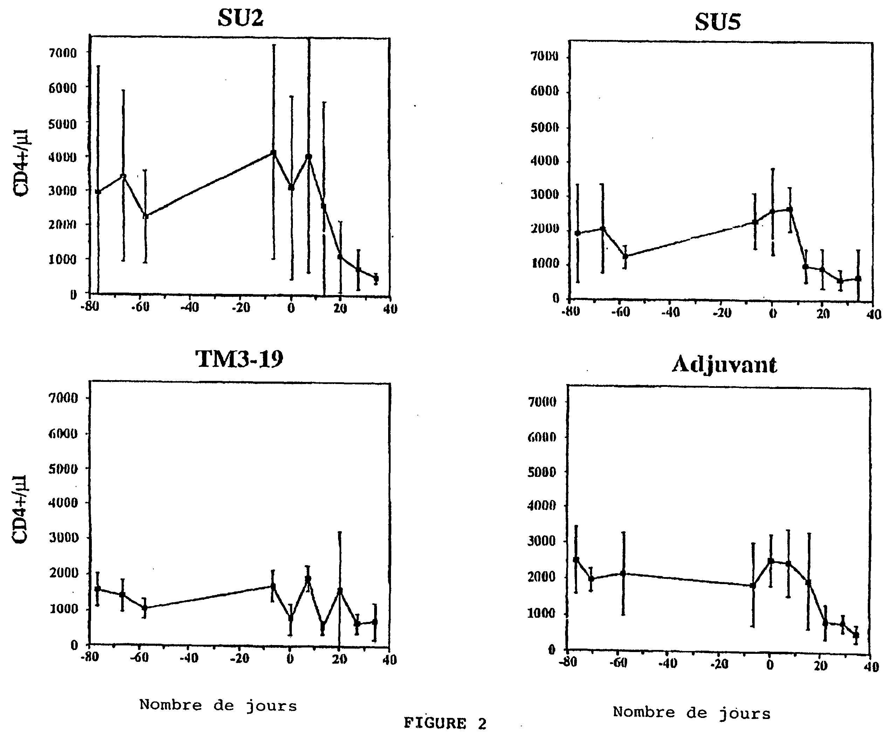

- la figure 2 correspond à une analyse des sous-ensembles de lymphocytes. Le nombre de lymphocytes CD4⁺ circulants, est exprimé sous la forme d'une valeur moyenne, pendant l'immunisation et après l'épreuve virulente, pendant l'infection aiguë ;

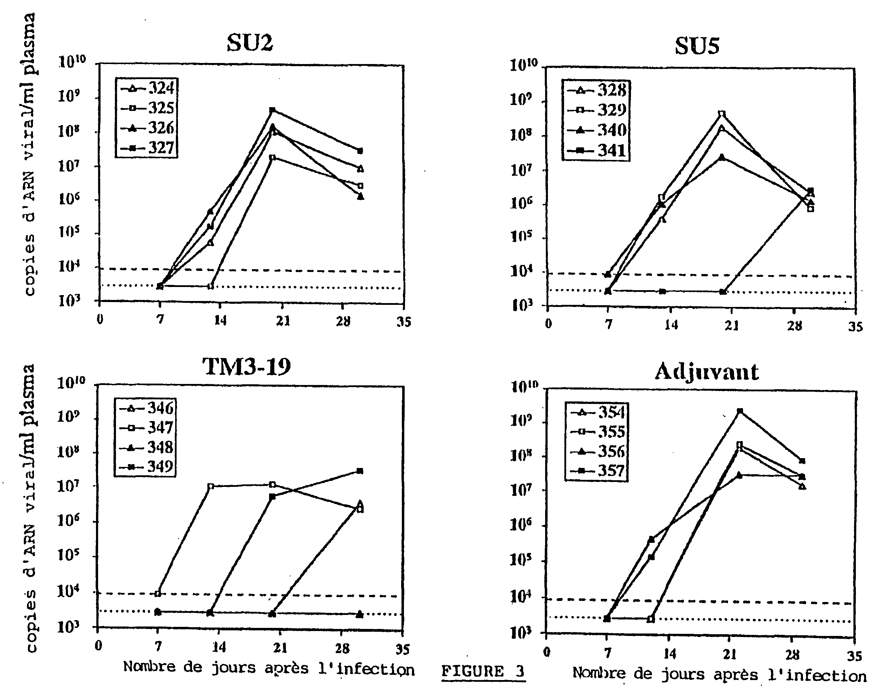

- la figure 3 illustre la charge virale dans le plasma lors de l'infection primaire. La charge virale, déterminée par RT-PCR est exprimée à l'aide du nombre de copies d'ARN viral/ml de plasma (ordonnées) en fonction du nombre de jours (abscisses). La ligne en traits discontinus correspond aux plasmas dans lesquels l'ARN viral, bien que détecté, est présent à des valeurs inférieures à 30 copies/réaction ( 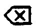 8580 copies/ml de plasma) ; la ligne en pointillés correspond au seuil de détection ( 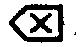 2 860 copies/ml de plasma) ;

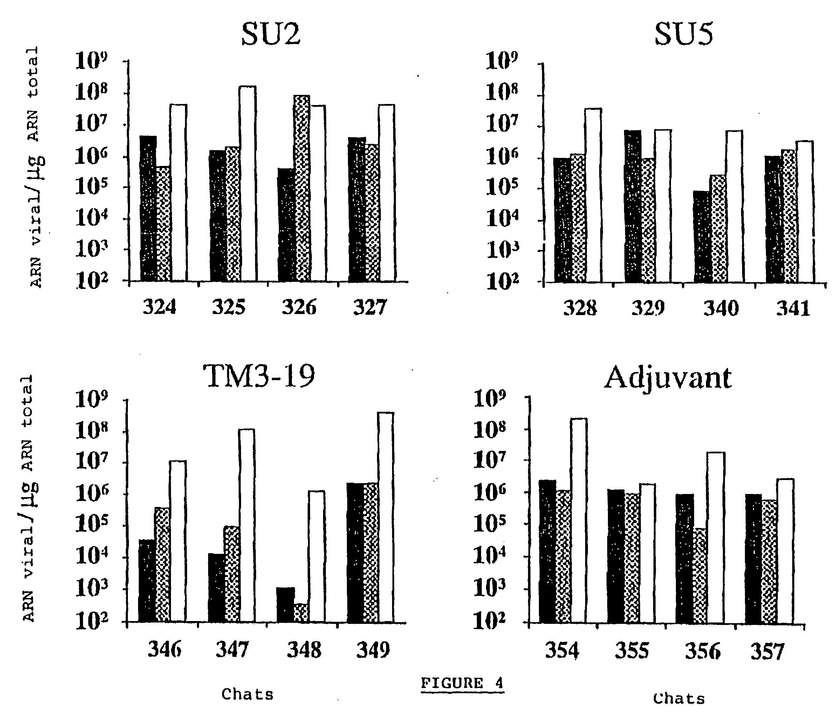

- la figure 4 illustre la charge virale dans les tissus. Elle est exprimée en nombre de copies d'ARN viral/µg d'ARN total (ordonnées), pour chaque chat. Elle est déterminée par RT-PCR. Les résultats obtenus sont illustrés pour les ganglions axillaires (■), la rate (□) et le thymus (□).
   Il doit être bien entendu, toutefois, que ces exemples sont donnés uniquement à titre d'illustration de l'objet de l'invention, dont ils ne constituent en aucune manière une limitation.

### EXEMPLE 1 : Matériel et méthodes mis en oeuvre pour illustrer l'activité protectrice des peptides selon l'invention.

### 1. Culture de tissu

Le clone ID 10 (Osborne R., *J. Gen. Virol.,* 1994, **75,** 3641-3645) de fibroblastes rénaux félins de Crandell (*Crandell's feline kidney cells* ou CrFK) est cultivé dans un milieu DMEM (*Dulbecco's modified Eagle's medium*) complémenté avec 10 % de sérum de veau foetal inactivé par la chaleur (*Fetal cal serum* ou FCS), 100 UI/ml de pénicilline et 100 µg/ml de streptomycine. La lignée cellulaire lymphoïde T féline FL-4 (Yamamoto J. et al., *Intervirology*, 1991, **32**, 361-375), qui est infectée de manière chronique, avec la souche Petaluma de VIF (Pedersen N.C. et al., *Science,* 1987, **235,** 790-793), est cultivée dans un milieu RPMI-1640 complémenté avec du FCS et les antibiotiques précités (milieu RPMI complet).

Les cellules mononucléées de sang périphérique félin (*Peripheral blood mononuclear cells* ou cellules PBM) sont isolées du sang de chats non infectés, par centrifugation sur gradient de densité et activées pendant 3 jours dans un milieu RPMI complet contenant 5 µg/ml de concanavaline A, 50 µM de 2-mercaptoéthanol (2-ME) et 10 mM d'HEPES.

### 2. Virus

Les isolats Petaluma (Pedersen N.C. et al., *Science,* 1987, **235,** 790-793) et Wo (Moraillon A. et al., *Vet. Microbiol.,* 1992, **31**, 41-54) de VIF sont obtenus, respectivement à partir de sumageants de la lignée cellulaire FL-4 et de cellules PBM félines infectées. L'isolat Wo a été soumis à un nombre limité de passages, uniquement sur cellules mononucléées (PBMC).

### 3. Peptides

Les peptides sont inclus dans les domaines de la glycoprotéine d'enveloppe définis antérieurement comme contenant des épitopes B continus (Pancino G. et al., *J. Virol*., 1993, **67**, 664-672).

Ils présentent les séquences présentées dans la liste des séquences, conformément au Tableau suivant :

| | |
|---|---|
| SU2 | SEQ ID NO:2 |
| SU5 | SEQ ID NO:3 |
| TM3-19 (759-777) | SEQ ID NO:1 |

La position de ces peptides dans la séquence *env* est illustrée à la figure 1. La numérotation correspond à celle de la séquence Wo (Pancino et al., *Virology,* 1993, **192**, 659-662).

### 4. Immunisation et épreuve virulente

Des groupes de 4 chats de 4 mois ½ sont immunisés par voie sous-cutanée avec 120 µg de peptide VIF (approximativement 250 µg de peptide couplé à la KLH (*keyhole limpet haemocyanin*)), émulsifié dans un adjuvant complet de Freund.

Quatre rappels sont administrés en sous-cutané dans un adjuvant de Freund incomplet, généralement à des intervalles de 2-3 semaines, bien que l'intervalle entre les 2 dernières injections ait été de 7 semaines.

Comme contrôle négatif, on utilise 4 chats ayant été soumis au même protocole d'injections en sous-cutané, avec un adjuvant uniquement.

Le développement de la réponse immune est suivi régulièrement.

Les chats sont soumis à une épreuve virulente, une semaine après la dernière immunisation, par une inoculation intrapéritonéale de 10 CID₅₀ d'isolat Wo de VIF, une CID₅₀ correspondant à la dose apte à infecter 50 % des chats.

On effectue sur le sang, recueilli chaque semaine, les tests suivants : énumération des lymphocytes T CD4⁺, isolement du virus, détermination de l'ARN viral dans le plasma (RT-PCR compétitive) et analyses sérologiques.

Les chats sont sacrifiés cinq semaines après l'épreuve virulente. Les organes lymphoïdes (rate, thymus, ganglions lymphatiques axiaux) sont récupérés et congelés dans de l'azote liquide, pour l'extraction de l'ARN.

### 5. Analyses sérologiques

La production d'anticorps est évaluée avant l'épreuve par un ELISA mettant en oeuvre un peptide immobilisé (SU2, SU5, TM2 ou TM3-19) et immunoprécipitation. Après l'épreuve, la séroconversion est surveillée par un ELISA mettant en oeuvre des peptides d'enveloppe immobilisés et une protéine de capside de VIF recombinante.

### 6. ELISA

L'ELISA avec les peptides d'enveloppe immobilisés correspondant aux domaines immunogéniques SU2 et TM2 et avec la protéine p25 recombinante (protéine de capside de VIF) immobilisée est réalisé comme décrit dans Avraméas A. et al. (*Res. Virol*., 1993, **144**, 209-218).

Des microplaques (Immunolon II, Dynatech) sont recouvertes avec 0,5 µg/puits pour les peptides et avec 0,1 µg/puits pour la protéine p25.

Les tests sont réalisés en double et les résultats sont exprimés en valeurs moyennes.

Les titres anti-peptides avant l'épreuve sont exprimés comme le logarithme de l'inverse de la dernière dilution de sérum fournissant une absorbance (405 nm) supérieure à 0,1.

Après l'épreuve, la séroconversion contre les peptides d'enveloppe et la protéine p25 est évaluée à des dilutions de sérum respectivement de 1/25 et de 1/100.

Les valeurs d'absorbance (405 nm) sont corrigées par soustraction des valeurs d'absorbance obtenues par liaison avec un peptide contrôle immobilisé.

Les essais sont normalisés en référence à la liaison d'un mélange de sérum de chats infectés par le VIF avec un peptide représentant le domaine immuno-dominant de la glycoprotéine transmembranaire de VIF (TM2) (Avraméas A. et al., *Res. Virol.,* 1993, **144,** 209-218).

### 7. Immunoprécipitation

Les sérums recueillis le jour de l'épreuve (à une dilution au 1/40) sont utilisés pour immunoprécipiter les glycoprotéines d'enveloppe dérivées des cellules FL-4 métaboliquement marquées et des cellules mononucléées félines (cellules PBM) infectées avec la souche Wo de VIF, comme décrit dans G. Pancino et al. (*Virology*, 1995, **206**, 796-806).

### 8. Tests de neutralisation et réduction d'infectivité virale

La détection des anticorps neutralisants l'infection des CrFK par la souche Petaluma de VIF est réalisée comme précédemment décrit (Richardson J. et al., *J. Gen. Virol.,* 1996, **77**, 759-771).

Pour le test de réduction de l'infectivité virale, des dilutions en série (1/50, 1/100, 1/200, 1/400) d'une suspension mère d'isolat Wo et une seule dilution de sérum (1/5) sont préparées dans un milieu RPMI complet contenant 50 µM de 2-ME et 10 mM d'HEPES.

Le sérum félin est mélangé avec un volume égal des dilutions en série de virus et incubé dans un volume final de 100 µl, pendant 1 heure à 37°C.

La concentration en cellules PBM activées par un mitogène est ajustée à 4.10⁶/ml dans un milieu RPMI complet comprenant du 2-ME, de l'HEPES et 200 U/ml d'IL-2 humaine recombinante. On ajoute aux tubes 0,1 ml de suspension cellulaire (4.10⁵ cellules).

Après une incubation d'une nuit, l'*inoculum* viral est récupéré en lavant les cellules 2 fois avec 0,5 ml de milieu RPMI complet.

Les cellules sont alors remises en suspension dans 0,2 ml de sérum félin dilué dans un milieu RPMI complet comprenant du 2-ME, de l'HEPES et 100 U/ml d'IL-2, puis transférées dans les puits de plaques de microtitration à 96 puits.

La moitié du milieu est remplacée 4 jours après l'infection.

Des aliquots de 10 µl sont récupérés 7 jours après l'infection pour une analyse de l'activité transcriptase inverse.

### 9. Analyse des sous-ensembles de lymphocytes

La numération des lymphocytes T CD4⁺ est effectuée à intervalles réguliers avant l'épreuve, le jour de l'épreuve et à intervalles réguliers pendant l'infection aiguë.

Les lymphocytes T CD4⁺ sont comptés par cytométrie de flux, en utilisant des anticorps monoclonaux (Clinisciences).

### 10. Isolement du virus

La virémie plasmatique est quantifiée par culture des cellules PBM (Ho D.D. et al., *N. Engl. J. Med*., 1989, **321**, 1621-1625).

L'infection est estimée par détermination de la p25 par ELISA (Petcheck, IDEXX) dans des surnageants de culture, obtenus 14 et 21 jours après l'inoculation de différents volumes de plasma (0,2, 2, 10, 40, 200 ou 1 000 µl). La dernière dilution plasmatique donnant une culture positive est considérée comme le point limite.

La virémie plasmatique est exprimée en TCID₅₀/ml de plasma (→doses aptes à infecter 50 % des cultures de tissus).

### 11. Préparation de l'ARN

### - ARN plasmatique

Le plasma est filtré (0,45 µm) et l'ARN non associé à des cellules est extrait directement du plasma filtré à l'aide du kit Viral RNA (Qiagene) et élué dans 50 µl d'eau selon les instructions du fabricant, puis l'ARN est aliquoté et stocké à -80°C.

### - ARN tissulaire

Les tissus de rate et de thymus congelés sont réduits en poudre et immédiatement dispersés dans le tampon de lyse fourni avec le kit d'extraction de l'ARN (RNeasy, Qiagène), avec l'aide d'un homogénéiseur tissulaire (Ultra-Turrax). Les ganglions lymphatiques axillaires congelés sont dispersés directement dans le tampon de lyse ; l'ARN est ensuite extrait en utilisant le kit RNeasy, selon les instructions du fabricant.

L'ADN contaminant est éliminé par hydrolyse avec de la DNase 1 (RQ1.RNase-free DNase, Promega).

### 12. RT-PCR compétitive

Une région conservée du gène gag du VIF est sélectionné comme séquence cible pour la transcription inverse de l'ARN compétitif et l'amplification PCR imbriquée.

L'amplification de la séquence cible native fournit un produit initial de 312 paires de bases et un produit imbriqué de 165 paires de bases, correspondant aux nucléotides 1059-1370 et aux nucléotides 1157-1321 du clone VIF 34TF10 (Talbott R.L. et al., Proc. Natl. Acad. Sci. USA, 1989, **86**, 5743-5747).

### - Constructions moléculaires

La séquence cible de 312 paires de bases est amplifiée à partir d'un plasmide (pKS*gag*) contenant tout le gène *gag* de la souche Wo de VIF (Pancino G. et al., *Virology*, 1993, **67**, 664-672), en utilisant comme amorce 5', la séquence SEQ ID NO:4 (SKWoG107), comprenant la séquence en 5' de la matrice cible et un site KpnI et comme amorce 3', la séquence SEQ ID NO:5 (RXWoG139), qui comprend la séquence en 3' de la matrice cible et un site XbaI, dans les mêmes conditions que celles exposées ci-après pour obtenir la séquence modifiée par délétion.

Le produit d'amplification est sous-cloné dans les sites correspondant du plasmide pBluescript KS+, aboutissant ainsi au plasmide pBSQC*gag*.

Pour préparer une matrice pour la synthèse de l'ARN de compétition, on supprime 31 nucléotides dans la séquence *gag*, par PCR.

La portion 3' de la séquence native est amplifiée en utilisant une amorce 5', SHΔWoG128 (SEQ ID NO:6), complémentaire du site naturel HindIII (nucléotides 1241-1246) et présentant une discontinuité de 31 nucléotides, et une amorce 3', RXWoG139 (SEQ ID NO:5), comprenant la séquence 3' de la matrice cible et un site XbaI, dans les conditions suivantes : la séquence native (10 ng de pBSQC*gag*), est amplifiée dans un volume final de 100 µl contenant 2,5 mM de MgCl₂, 200 µM de dNTP, 1x de tampon commercial et 2,5 U de Taq polymérase (GibcoBRL) et chaque amorce à 0,2 µM. L'ADN est dénaturé à 94°C pendant 3 min, soumis à 30 cycles d'amplification (94°C 30 s/55°C 30 s/72°C 30 s) et soumis à une élongation à 72°C pendant 7 min, en présence des amorces de séquence SEQ ID NO:6 et SEQ ID NO :5.

Le produit d'amplification est purifié, digéré par les enzymes HindIII et XbaI et substitué au fragment correspondant de pBSQC*gag*, conduisant au plasmide pBSQCΔ*gag*.

### - Synthèse de l'ARN de compétition

L'ARN de compétition est synthétisé à partir du produit de transcription du plasmide pBSQCΔ*gag* linéarisé par XbaI, en utilisant la T3 RNA polymérase (Proméga Gemini kit).

La matrice d'ADN est hydrolysée avec la RQ1 Rnase-free DNase (Proméga). L'ARN de compétition est purifié par absorption sur de la silice (Rneasy, Qiagène) et quantifié par mesure de l'absorbance à 260 nm. L'ARN est aliquoté et stocké à -80°C.

### - RT-PCR compétitive

Pour la synthèse de l'ADNc, 2,5 µl d'ARN viral sont mélangés avec 2,5 µl contenant différents nombres de copies d'ARN de compétition ; de manière typique, on prépare les différents échantillons d'ARN de compétition à partir de 5 dilutions semi-logarithmique, encadrant le nombre de copies estimé dans l'échantillon biologique.

L'ARN est dénaturé à 65°C pendant 5 min et immédiatement placé sur de la glace. Les réactifs pour la transcription inverse (15 µl) sont ajoutés.

Les mélanges de réactions finals contiennent 0,3 U/ml d'hexanucléotides aléatoires (séquences de 6 nucléotides alignés aléatoirement qui permettent d'amplifier théoriquement tout fragment d'ARN) (Pharmacia), 0,5 mM de dNTP, 10 mM de DTT, 1x de tampon commercial (Gibco BRL) et 100 U de Superscript II (Gibco BRL), dans un volume de 20 µl.

Les réactions sont d'abord réalisées à 25°C pendant 10 min pour favoriser l'hybridation des amorces, puis à 42°C pendant 50 min. La transcriptase inverse est inactivée par incubation à 95°C pendant 5 min.

Comme témoin, tous les échantillons d'ARN sont aussi amplifiés directement par PCR pour vérifier la complète élimination de l'ADN éventuellement présent dans l'échantillon biologique.

Les séquences hautement conservées sont sélectionnées comme amorces. L'ADN complémentaire est amplifié par PCR imbriquée, en utilisant :
. comme amorces externes : et
. comme amorces internes :

Pour la première amplification, les réactifs de PCR (80 µl) sont ajoutés en mélange à 20 µl d'ADNc.

De manière plus précise, le mélange réactionnel contient : 200 µM de dNTP, 1,5 mM de MgCl₂, 0,1 µM de chaque amorce externe, 0,8x de tampon commercial (Gibco BRL) et 0,25 U de Taq polymérase.

L'ADN est dénaturé à 94°C pendant 3 min, soumis à 28 cycles d'amplification (94°C 30 s/55°C 30 s/72°C 30 s) et à une élongation à 72°C pendant 7 min.

Pour l'amplification imbriquée, 2 µl du produit de la première amplification est transféré dans 98 µl de mélange réactionnel contenant 200 µM de dNTP, 1,5 mM de MgCl₂, 0,5 µM de chaque amorce interne, 1x de tampon commercial (Gibco BRL) et 0,25 U de Taq polymérase (Gibco BRL).

L'ADN est dénaturé à 94°C pendant 3 min, soumis à 28 cycles d'amplification (94°C 30 s/52°C 30 s/72°C 30 s) et à une élongation à 72°C pendant 7 min.

### - Analyse

Les produits d'amplification (10 µl) sont soumis à une électrophorèse sur gel d'agarose à 2,75 %.

Les images digitales des gels colorés au bromure d'éthidium sont obtenues à l'aide de l'Imager d'Appligène et les analyses densitométriques sont réalisées avec le logiciel Image NIH. La densité du produit de compétition est ajustée en fonction de la différence entre la longueur de la séquence native et de la séquence de compétition.

Le logarithme du rapport densité de produit de compétition/densité du produit natif est tracé en fonction du logarithme du nombre de copies d'ARN de compétition ajouté à chaque réaction.

Le meilleur ajustement est déterminé par la méthode des moindres carrés et le nombre de copies d'ARN natif correspond à l'intersection de l'axe des X (Piatak M. Jr. et al., Biotechniques, 1993, 14, 70-80).

### 13. Analyse statistique

La surface cumulée sous la courbe (Dawson J., Drug Information J., 1994, **28**, 723-732) est calculée à partir des graphiques illustrant le rapport copies d'ARN viral/ml de plasma/temps et comparée par le test de Mann-Whitney.

### EXEMPLE 2 : Évaluation de la réponse humorale obtenue avec TM3-19 et de ses effets de protection.

### 1. Développement d'une réponse humorale à l'encontre des peptides d'enveloppe.

Pour évaluer l'influence de l'immunité humorale vis-à-vis de domaines particuliers des glycoprotéines d'enveloppe lors d'une infection primaire à VIF, des chats sont immunisés avec des peptides (figure 1) correspondant aux domaines d'enveloppe comprenant les épitopes B continus reconnus lors de l'infection naturelle.

Tous les chats immunisés développent une forte réponse en anticorps, comme déterminé en ELISA avec des peptides d'enveloppe utilisés comme immunogènes.

Les résultats de ces ELISA sont résumés dans le Tableau I ci-après :

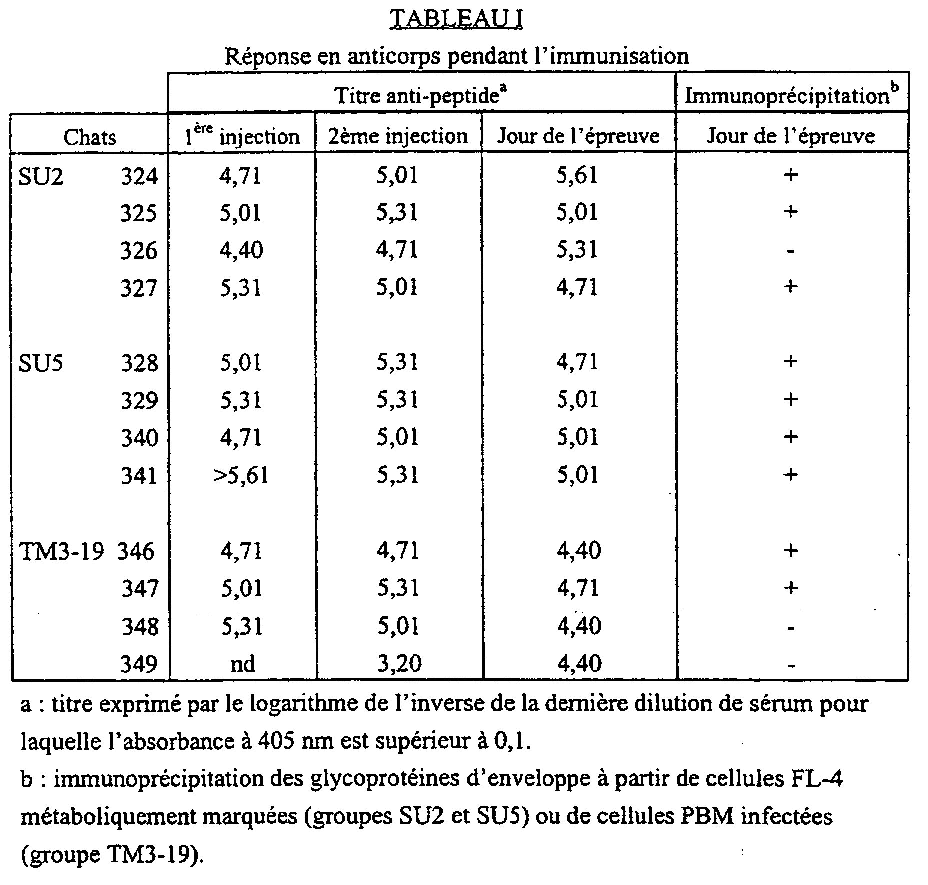

Le jour de l'épreuve, les titres, exprimés par l'inverse du logarithme de la dilution de sérum, sont compris entre 4,40 et 5,61.

La reconnaissance des glycoprotéines d'enveloppe est estimée par immunoprécipitation de la glycoprotéine d'enveloppe marquée biosynthétiquement avec des sérums recueillis le jour de l'épreuve, comme résumé au Tableau I.

Les sérums de tous les chats immunisés avec le peptide SU5 et 3 des 4 chats (324, 325 et 327) immunisés avec le peptide SU2 immunoprécipitent les glycoprotéines d'enveloppe de la souche Petaluma obtenues à partir de lysats de cellules FL-4 infectées de manière chronique.

Dans la mesure où le peptide TM3-19, déduit de la séquence Env du VIF Wo, comprend 4 résidus différents sur 19, par rapport à la séquence Petaluma, la capacité d'immunoprécipiter des sérums avant l'épreuve des chats immunisés par le peptide TM3-19 a été testé contre les glycoprotéines d'enveloppe de la souche Wo, obtenues à partir de lysats de cellules PBM après infection aiguë par le VIF Wo.

Tandis que seulement les sérums de 2 chats (346, 347) immunisés avec le peptide TM3-19 précipitent la protéine d'enveloppe de la souche Wo, l'immunoprécipitation de l'enveloppe virale est moins efficace à partir des cellules PBM infectées en aigu qu'à partir des lignées cellulaires lymphoblastoïdes infectées de manière chronique.

### 2. Activité des anticorps dirigés contre les peptides d'enveloppe dans un test biologique.

Pour évaluer la fonction des anticorps, les sérums félins dirigés contre le peptide SU2 sont testés pour leur valeur neutralisante vis-à-vis de l'infection des CrFK par la souche Petaluma de VIF.

Seulement un chat immunisé (327) par le peptide SU2 développe des anticorps neutralisants : la dernière dilution réduisant l'infection d'au moins 50 % est 1/160.

Les sérums des chats immunisés avec les peptides SU5 et TM3-19 ne neutralisent pas le VIF Petaluma (mesure dans la lignée cellulaire CrFK).

En raison de la diversité de la séquence des glycoprotéines d'enveloppe des souches Petaluma et Wo, et en particulier au niveau du domaine TM3, l'activité fonctionnelle des anticorps a également été étudiée dans un test homologue.

Dans la mesure où la souche Wo n'a pas été adaptée au passage sur cellules CrFK, l'influence des anticorps a été étudiée sur l'infection des cellules PBMC félines.

Dans un test de réduction d'infectivité utilisant des cellules PBMC activées par un mitogène, les sérums dirigés contre les peptides SU2 et TM3-19 ne modifient pas l'infection par VIF Wo, en dépit de l'activité neutralisante qui a été observée pour l'un des sérums dirigé contre le peptide SU2, lorsque des cellules CrFK sont utilisées comme substrat cellulaire.

### 3. Isolement du VIF après l'épreuve virulente.

Après l'épreuve, tous les chats sont infectés, comme déterminé par isolement du VIF dans des cellules PBM, après inoculation du plasma recueilli, 2 et 3 semaines après l'épreuve.

### 4. Séroconversion après épreuve virulente.

Après épreuve avec la souche Wo de VIF, la plupart des chats présentent une réponse humorale vis-à-vis des composants viraux, comme illustré par l'ELISA avec les peptides SU2 et TM2, dérivés des glycoprotéines externes et transmembranaires, respectivement et la protéine de capside recombinante.

Les résultats sont illustrés au Tableau II ci-après.

**TABLEAU II**

| Liaison des sérums aux composants viraux en fonction du temps (semaines) après l'épreuve virulente^{a} | | | | | | | | | | |
|---|---|---|---|---|---|---|---|---|---|---|
| Chats | | SU2^{b} | | | TM2 | | | p25 | | |
| Groupe | Chat n° | 3 | 4 | 5 | 3 | 4 | 5 | 3 | 4 | 5 |
| SU2 | 324 | - | - | - | 0 | 2,1 | 2,5 | 0 | 0,2 | 1,9 |
| | 325 | - | - | - | 0 | 1,4 | 2,1 | 0 | 0 | 0,2 |
| | 326 | - | - | - | 0 | 1,3 | 2,1 | 0 | 0,2 | 1,1 |
| | 327 | - | - | - | 0 | 2,2 | 2,3 | 0 | 0,4 | 1,3 |
| SU5 | 328 | 0 | 1,9 | 2,4 | 2,3 | 2,9 | 3,0 | 0,1 | 2,7 | 2,9 |
| | 329 | 0 | 0,5 | 0,7 | 0 | 2,8 | 3,1 | 0 | 1,0 | 2,2 |
| | 340 | 0 | 0 | 0,1 | 0,1 | 2,4 | 2,8 | 0 | 0,2 | 0,8 |
| | 341 | 0 | 0 | 0 | 0 | 0 | 0 | 0 | 0 | 0 |
| TM3-19 | 346 | 0 | 0 | 0 | 0 | 0,1 | 1,9 | 0 | 0,1 | 0,6 |
| | 347 | 0 | 0 | 0 | 1,2 | 2,4 | 2,5 | 0,2 | 2,3 | 2,3 |
| | 348 | 0 | 0 | 0 | 0 | 0 | 0 | 0 | 0 | 0 |
| | 349 | 0 | 0 | 0 | 0 | 0,5 | 2,3 | 0,1 | 0 | 0,1 |
| Adjuvant | 354 | 0 | 2,4 | 3,0 | 0 | 2,6 | 3,0 | 0 | 0 | 2,6 |
| | 355 | 0 | 0,2 | 0,2 | 0,1 | 2,3 | 3,1 | 0 | 0,1 | 2,0 |
| | 356 | 0,4 | 0,3 | 0,3 | 2,2 | 3,0 | 3,1 | 0 | 0,5 | 1,4 |
| | 357 | 0 | 0,2 | 1,0 | 0,2 | 2,9 | 3,1 | 0, | 0,9 | 2,5 |

| | | | | | | | | | | |
|---|---|---|---|---|---|---|---|---|---|---|
| ^{a} données obtenues par ELISA mis en oeuvre avec les peptides d'enveloppe SU2 et TM2 et la protéine de capside de VIF recombinante p25 pour des dilutions sériques de 1/25 (peptides) et de 1/100 (p25) ; ces données sont exprimées en densité optique (voir exemple 1) ; | | | | | | | | | | |
| ^{b} données non illustrées pour le groupe de chats vacciné avec SU2 (séropositifs avant l'épreuve virulente). | | | | | | | | | | |

Le développement d'une réponse humorale anti-virale est plus faible et retardée chez 3 chats (346, 348, 349) immunisés avec le peptide TM3-19, en comparaison avec la réponse des chats contrôles, quoique le dernier chat (347) de ce groupe a développé une réponse en anticorps forte et rapide.

5 semaines après l'épreuve, aucun des chats immunisé avec le peptide TM3-19 n'a développé une réponse en anticorps détectable vis-à-vis du peptide SU2, tandis que les chats contrôles présentent des réponses détectables.

Alors qu'à 4 semaines la plupart (11/12) des chats immunisés avec les peptides SU2 et SU5 et les chats ayant reçu uniquement de l'adjuvant développent une réponse importante contre le peptide TM2, tous les chats sauf un (347) immunisés avec le peptide TM3-19 présentent une réponse faible ou indétectable.

Alors qu'à 5 semaines, tous les chats contrôles répondent bien à la protéine p25, un chat (348) immunisé avec le peptide TM3-19 ne répond pas et parmi les 3 autres chats, 2 animaux (346, 349) n'ont développé que de faibles réponses.

### 5. Modifications hématologiques pendant l'infection aiguë.

Pour évaluer l'effet de l'immunisation sur la pathologie associée à une infection primaire, les lymphocytes circulants CD4⁺ sont comptés avant et après l'épreuve avec la souche Wo du VIF.

Le nombre de lymphocytes CD4⁺ est illustré à la figure 2.

Quoique les tendances soient masquées par une hétérogénéité individuelle, la plupart des chats immunisés avec les peptides SU2 et SU5 ainsi que les chats qui n'ont reçu que de l'adjuvant, présentent une réduction progressive du nombre de lymphocytes CD4⁺ après l'infection (figure 2).

Malgré un certain degré de fluctuation, le nombre de lymphocytes CD4⁺ ne montrent pas une diminution importante comme celle observée chez les autres groupes lors de l'infection aiguë chez les chats du groupe TM3-19 (figure 2).

### 6. Charge virale plasmatique déterminée par RT-PCR

Dans la mesure où l'on ne pouvait pas s'attendre à obtenir une immunité importante par vaccination avec un seul domaine des glycoprotéines d'enveloppe, il était nécessaire de mettre au point une méthode quantitative d'évaluation de la charge virale qui devait permettre la détection de réductions modestes, mais ayant une signification particulièrement importante, de la dissémination virale.

C'est pourquoi les Inventeurs ont mis au point une RT-PCR compétitive qui a permis la détermination quantitative de l'ARN viral dans le plasma et les tissus.

Cet essai permet la détection de 10 copies d'ARN, lors d'une réaction, ce qui établit la limite inférieure de détection à 1430 copies d'ARN/ml de plasma lorsque l'ARN viral est extrait à partir de 140 µl de plasma.

Après épreuve avec 10 CID₅₀ d'isolat Wo, l'ARN viral est détectable dans le plasma par RT-PCR, deux semaines après l'infection.

Chez les chats qui ont reçu uniquement de l'adjuvant, on observe un pic du nombre de copies d'ARN viral, à trois semaines, qui peut atteindre jusqu'à 2,5x10⁹ copies/ml de plasma (figure 3).

La présence d'anticorps dirigés contre un domaine connu pour induire des anticorps neutralisants, SU2, avant l'épreuve n'apparaît pas modifier le cours de l'infection primaire.

Parmi les chats immunisés avec le peptide SU5, l'infection aiguë de 3 chats a suivi une cinétique similaire à celle des chats contrôles, tandis que l'infection chez l'un des chats apparaît de manière retardée.

Le cours de l'infection chez les chats immunisés avec le peptide TM3-19 est généralement différent de celui observé avec les chats contrôles.

Quoique l'infection chez l'un des chats (347) apparaît rapidement, l'infection chez les 3 autres chats de ce groupe apparaît de manière retardée.

En outre, il n'a pas été possible de détecter une virémie plasmatique chez l'un des chats (348) pendant la période de mesure.

Le nombre de copies d'ARN viral observé chez les chats immunisés avec le peptide TM3-19 n'excède jamais 3,1x10⁶ copies/ml, ce qui indique une diminution significative par rapport à la virémie observée dans le groupe de chats contrôles.

Ces données ont été analysées en comparant l'aire sous la courbe cumulée (Dawson J.D., *Drug Information J.,* 1994, **28,** 723-732) à 3 et 4 semaines après l'épreuve.

Avec le test de Mann-Whitney, la réduction de la charge virale observée chez les chats immunisés avec le peptide TM3-19 par rapport aux chats contrôles est significative aussi bien à 3 semaines qu'à 4 semaines (p = 0,02).

### 7. Virémie plasmatique

Pour les chats immunisés avec le peptide TM3-19 et les chats contrôles, la charge virale plasmatique est évaluée 3 semaines après l'épreuve par isolement du virus.

Le nombre de copies d'ARN viral déterminé par RT-PCR compétitive est comparé avec la virémie plasmatique déterminée par culture des cellules PBM.

Les résultats sont illustrés au Tableau III ci-après.

**TABLEAU III**

| | | Virémie plasmatique | |
|---|---|---|---|
| | Chats | RT-PCR^{α} | Culture PBMC^{β} |
| TM3-19 | 346 | indétectable | 1 |
| | 347 | 1,19 x 10⁷ | 1 x 10² |
| | 348 | indétectable | 1 |
| | 349 | 5,56 x 10⁶ | 1 |
| Adjuvant | 354 | 1,93 x 10⁸ | 5 x 10³ |
| | 355 | 2,43 x 10⁸ | 5 x 10² |
| | 356 | 3,12 x 10⁷ | 5 x 10³ |
| | 357 | 2,54 x 10⁹ | 5 x 10⁴ |

| | | | |
|---|---|---|---|
| ^{α} : la virémie plasmatique est mesurée par RT-PCR compétitive et exprimée en nombre de copies d'ARN viral/ml de plasma. | | | |
| ^{β} : la virémie plasmatique est mesurée par culture de cellules PBM et exprimée en TCID₅₀/ml de plasma | | | |

L'immunisation avec le peptide TM3-19 avant l'épreuve virulente entraîne une réduction importante (environ 3 logs) du taux de virus infectieux dans le plasma, de ¾ chats, très tôt au cours de l'infection primaire.

Ces données confirment que les taux réduits d'ARN viral observés pour les chats immunisés avec le peptide TM3-19 en comparaison avec ceux obtenus avec les chats contrôles reflètent bien la réduction du titre plasmatique du virus infectieux.

### 8. Charge virale dans les tissus, déterminée par RT-PCR compétitive.

La charge virale dans les tissus est évaluée 5 semaines après l'épreuve par numération du nombre de copies d'ARN viral dans les ganglions lymphatiques axillaires et le thymus par RT-PCR compétitive.

Dans la mesure où le fragment amplifié (amplicon) est localisé dans l'intron de l'ARN viral sous-génomique, les copies d'ARN viral dénombrées dans les tissus, représentent les transcrits complets, soit intracellulaires, soit encapsidés dans les particules virales extracellulaires.

L'ARN viral est détecté dans tous les tissus examinés et chez tous les chats (figure 4).

Les taux d'ARN viral dans les ganglions lymphatiques axillaires sont plus faibles chez trois chats (346, 348 et 349) immunisés avec le peptide TM3-19 que chez les chats immunisés avec les peptides SU2 et SU5 et les chats contrôles.

Dans la rate, le taux d'ARN viral est particulièrement faible chez un chat (348) immunisé avec le peptide TM3-19.

Dans le thymus, cependant, la charge virale est similaire dans les 4 groupes de chats, et de manière générale, elle est plus élevée que dans les autres tissus examinés.

Une charge virale élevée dans le thymus, à une phase précoce de l'infection a également été observée par hybridation *in situ* (Beebe A.M. et al., *J. Virol*., 1994, **68**, 3080-3091).

Il n'est pas possible de savoir si l'infection apparente plus importante du thymus reflète (i) sa richesse en cellules infectées de manière préférentielle en début d'infection, (ii) une capacité importante à piéger les particules virales, ou (iii) dans la nature séquentielle de la colonisation (tissus examinés avant la résolution de l'infection aiguë).

Ces résultats montrent qu'alors que l'immunisation avec deux peptides dérivés de la glycoprotéine de surface (SU2 et SU5) ne modifient pas le cours de l'infection, l'immunisation avec le peptide TM3-19 dérivé du domaine proximal membranaire extracellulaire de la glycoprotéine transmembranaire retarde de manière significative la dissémination virale.

Ces résultats montrent également que l'induction d'anticorps qui neutralisent l'infection virale *in vitro* ne présente pas forcément une bonne corrélation avec la protection immunitaire recherchée.

En particulier, la corrélation activité neutralisante/protection est problématique, notamment dans la mesure où l'activité mesurée dépend des conditions de l'essai utilisé et plus particulièrement du substrat cellulaire utilisé pour mesurer l'infectivité résiduelle.

En fait, la neutralisation des isolats lentiviraux primaires est fondamentalement différente de la neutralisation du virus adapté à une propagation dans des cultures cellulaires.

Les résultats obtenus avec les peptides selon l'invention montrent également qu'ils retardent de manière significative l'infection.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Utilisation du peptide de SEQ ID NO:1 pour préparer un médicament pour le traitement ou la prévention des infections à VIF.

2. Peptide, **caractérisé en ce qu'**il est défini par la SEQ ID NO:1.

3. Composition immunoprotectrice contre une infection à VIF, **caractérisée en ce qu'**elle est essentiellement constituée par au moins le peptide de SEQ ID NO:1 selon la revendication 2, et au moins un véhicule pharmaceutiquement acceptable.

4. Composition selon la revendication 3, pour son utilisation dans la prévention des infections à VIF.

5. Composition selon la revendication 3, pour son utilisation dans le traitement des infections à VIF.

## Patentansprüche

1. Verwendung des Peptids der SEQ ID NO:1 zur Herstellung eines Medikaments für die Behandlung oder die Prävention von Infektionen mit FIV.

2. Peptid, **dadurch gekennzeichnet, dass** es durch SEQ ID NO:1 definiert ist.

3. Immunprotektive Zusammensetzung gegen eine Infektion mit FIV, **dadurch gekennzeichnet, dass** sie im Wesentlichen aus wenigstens dem Peptid der SEQ ID NO:1 nach Anspruch 1 und wenigstens einem pharmazeutisch annehmbaren Vehikel besteht.

4. Zusammensetzung nach Anspruch 3 zur Verwendung in der Prävention von Infektionen mit FIV.

5. Zusammensetzung nach Anspruch 3 zur Verwendung in der Behandlung von Infektionen mit FIV.

## Claims

1. Use of a peptide of SEQ ID NO:1 to prepare a medicinal product for the treatment or prevention of infections with FIV.

2. Peptide, **characterized in that** it is defined by SEQ ID NO:1.

3. Composition which provides immunoprotection against an infection with FIV, **characterized in that** it consists essentially of at least the peptide of SEQ ID NO:1 according to claim 2, and at least one pharmaceutically acceptable vehicle.

4. Composition according to claim 3, for its use in the prevention of infections with FIV.

5. Composition according to claim 3, for its use in the treatment of infections with FIV.


### LISTAGE DE SEQUENCE

<110> RICHARDSON, Jennifer
   MORAILLON, Anne
   SONIGO, Pierre
   PANCINO, Gianfranco
   CENTRE NATIONAL DE LA RECHERCHE SCIENTIFIQUE
<120> Peptides issus du gène env du virus de l'immunodéficience féline et leurs applications immunoprotectrices et vaccinales
<130> Sample file reference
<140>
   <141>
<150> FR 9713042
   <151> 1997-10-17
<160> 10
<170> PatentIn Vers. 2.0
<210> 1
   <211> 19
   <212> PRT
   <213> Feline immunodeficiency virus
<400> 1
   Lys Lys Gly Leu Gln Gln Leu Gln Glu Trp Glu Asp Trp Val Gly Trp
   1 5 10 15
   Ile Gly Asn
<210> 2
   <211> 17
   <212> PRT
   <213> Feline immunodeficiency virus
<400> 2
   Arg Ala Ile Ser Ser Trp Lys Gln Arg Asn Arg Trp Glu Trp Arg Pro
   1 5 10 15
   Asp
<210> 3
   <211> 22
   <212> PRT
   <213> Feline immunodeficiency virus
<400> 3
   Gln Val Val Lys Gln Pro Asp Tyr Leu Val Val Pro Gly Glu Val Met
   1 5 10 15
   Glu Tyr Lys Pro Arg Arg
   20
<210> 4
   <211> 35
   <212> ADN
   <213> Feline immunodeficiency virus
<400> 4
   cggggtaccc aatatgtagc acttgaccca aaaat 35
<210> 5
   <211> 34
   <212> ADN
   <213> Feline immunodeficiency virus
<400> 5
   gctctagatc ttgcttctgc ttgttgttct tgag 34
<210> 6
   <211> 34
   <212> ADN
   <213> Feline immunodeficiency virus
<400> 6
   ggatgaaagc ttaaagcccc tgatggtcct agac 34
<210> 7
   <211> 26
   <212> ADN
   <213> Feline immunodeficiency virus
<400> 7
   caatatgtag cacttgaccc aaaaat 26
<210> 8
   <211> 26
   <212> ADN
   <213> Feline immunodeficiency virus
<400> 8
   tcttgcttct gcttgttgtt cttgag 26
<210> 9
   <211> 26
   <212> ADN
   <213> Feline immunodeficiency virus
<400> 9
   ctctgcaaat ttaacaccta ctgaca 26
<210> 10
   <211> 26
   <212> ADN
   <213> Feline immunodeficiency virus
<400> 10
   gctgcagtaa aatagggtaa tggtct 26